# FASCICULE DE BREVET EUROPEEN

(11) **EP 1 674 122 B1**
(45) Date de publication et mention de la délivrance du brevet: **12.12.2012**
(21) Numéro de dépôt: 05301067.4
(22) Date de dépôt: 16.12.2005
(51) Int. Cl.: A61M 15/00

(54) **Appareil pour l'administration d'un aérosol médicamenteux**
Vorrichtung zum Verabreichen eines medizinischen Aerosols
Device for administering a medicinal aerosol

(30) Priorité: 21.12.2004 FR 0413784
(43) Date de publication de la demande: 28.06.2006
(73) Titulaire: LA DIFFUSION TECHNIQUE FRANCAISE, 42000 Saint-Etienne (FR)
(72) Inventeur: Vecellio-None, Laurent, 37170, Chambray les Tours (FR); Chantrel, Gilles, 42100, Saint-Etienne (FR); Massardier, Michel, 42000, Saint-Etienne (FR)
(74) Mandataire: Dupuis, François

(56) Documents cités:
- WO-A-03/089036
- US-A- 5 178 138
- US-A- 5 297 543
- US-A- 5 596 982
- US-A- 6 070 573

## Description

L'invention se rattache au secteur technique des systèmes de génération d'aérosols médicaux.

Les systèmes de génération d'aérosols médicaux ont pour fonction de transformer, un liquide ou une poudre, médicamenteux sous forme d'aérosol pour être administré dans les voies aériennes respiratoires.

Divers systèmes de génération d'aérosols médicaux existent sur le marché avec des sous formes d'appareils à commandes pneumatiques, ultra soniques, à membrane vibrante, notamment, ainsi que des flacons pressurisés avec valve doseuse.

Le Déposant est l'un des leaders dans la fabrication et la commercialisation de ce type d'appareils. Malgré tous les efforts de recherche en rapport avec ces appareils, générateurs, nébuliseurs, flacons pressurisés, les différentes études publiées en rapport sont toutes d'accord pour constater qu'une large part de l'aérosol projeté est perdue, gaspillée et ne profite pas au traitement thérapeutique souhaité. La part d'utilisation de l'aérosol dans sa fonction est ainsi estimée par les auteurs et fabricants à 25 % environ, ce qui est couramment explicité par la fraction inhalable ou disponible. La déperdition est due à plusieurs paramètres avec la perte de médicament dans l'atmosphère durant la phase expiratoire du patient, par la masse de médicament perdu dans le nébuliseur en fin de nébulisation, par la masse perdue en cours de transfert de l'aérosol. La conception des appareils avec des ouies de sorties en est une explication, les particules allant naturellement à l'air libre.

Les chambres d'inhalation, par exemple dans le cas des flacons pressurisés avec valve sont un moyen de limiter la déperdition.

Dans ce cas, le transfert de l'aérosol s'effectue essentiellement dans un réservoir qui se trouve à l'horizontal lorsque l'appareil est en bouche, de sorte que l'aérosol projeté qui est extrêmement variable et hétérogène dans la constitution de ses particules remplit partiellement le volume considéré. Ainsi, on constate sans difficultés que les particules de taille importante ont tendance à tomber très rapidement dans le fond du réservoir horizontal bien avant l'inhalation de par l'effet de simple gravité. On constate ainsi sans difficultés l'existence d'une fine couche de particules médicamenteuses non inhalées.

Certains autres appareils en particulier avec membrane vibrante notamment permettent la projection de l'aérosol dans son intégralité, dans une chambre verticale de petite dimension, mais la partie inférieure de cette chambre formant réservoir est susceptible de recevoir des particules médicamenteuses avec une vitesse élevée provoquant une impaction et donc une perte des particules médicamenteuses sur les parois de la chambre. En toute hypothèse, et selon les essais ainsi effectués, même pour ce type d'appareils, l'utilisation active de l'aérosol projeté est en moyenne de 25 % du volume introduit dans le système de génération.

Selon l'art antérieur, tel que défini par exemple dans les brevets US 5596982 et WO 03/089036, les appareils générateurs d'aérosols mettent en oeuvre lors du transport de l'aérosol des effets de turbulence ou effet Vortex qui sont à l'origine de pertes de particules d'aérosols. L'obtention de cet effet tourbillonnaire requiert que la pénétration de l'air s'effectue dans un plan perpendiculaire à l'axe de transport de l'aérosol.

Le document US 6 070 573 décrit un appareil pour l'administration d'un aérosol médicamenteux comprenant une chambre d'inhalation cylindrique qui utilise la gravité afin de séparer les particules d'aérosol selon leur taille, les particules de grosse taille se déposant sur la paroi de fond de la chambre d'inhalation.

L'objectif de l'invention a été de réduire la perte d'aérosols.

La démarche du Demandeur a donc été de reconsidérer le problème de cette utilisation partielle d'aérosol et de rechercher une solution pour améliorer ainsi les conditions de traitement thérapeutique des patients.

Différentes possibilités ont été ainsi examinées en réduisant ou supprimant la ou les ouies ou ouvertures établies sur le nébuliseur, ou l'introduction d'un système de soufflerie complémentaire pour augmenter la circulation de l'aérosol médicamenteux dans la chambre horizontale de transfert vers la zone d'inhalation buccale.

En pratique, ces solutions ne peuvent être retenues car répondant partiellement au problème posé améliorant d'une manière peu significative la proportion d'aérosol restant inhalé.

Face à cette situation, le Demandeur s'est alors orienté sur une conception différente de ce type d'appareils qui de manière inattendue apporte des propriétés de conservation de l'aérosol et de son inhalation dans des proportions sans aucune mesure avec ce qui est connu de l'art antérieur, puisque les premiers essais et tests valorisent la proportion d'aérosol inhalé entre 40% et 90 % comparativement aux 25 % précités.

L'objet de la présente invention consiste en un appareil pour l'administration d'un aérosol médicamenteux tel que défini dans la revendication 1. Cet appareil utilise un dispositif de transfert d'aérosol qui

permet de limiter la perte d'aérosol par dépôt sur les parois. En effet, dans le cadre des aérosols médicaux, les particules qui les constituent ont un diamètre de l'ordre du micromètre afin d'assurer leur pénétration et leur dépôt dans les voies respiratoires. Dans cette gamme de diamètre, la force de gravité s'applique sur les particules. Les particules sous l'action de la pesanteur ont une trajectoire dans une direction verticale et dans un sens vers le bas. Dans ces conditions, les particules tombent sous l'effet de leur propre poids (sédimentation) jusqu'à ce qu'elles rencontrent un obstacle pour s'y déposer. L'invention consiste à utiliser un volume vertical pour stocker l'aérosol afin de limiter le dépôt des particules par sédimentation. En introduisant un aérosol par le haut d'un volume vertical pour stocker l'aérosol, les particules mettront plus de temps à se déposer sur le fond du moyen complémentaire qu'avec un volume équivalent mais profilé dans un plan horizontal. Ce moyen complémentaire combiné avec la position relative des ouvertures d'entrée d'air et de sortie d'aérosol permet donc d'augmenter la quantité d'aérosol stocké entre chaque inhalation et de limiter son dépôt sur les parois. Il permet également de recevoir la génération d'un aérosol avec une vitesse élevée sans perte sur les parois. Le volume dans lequel est projeté l'aérosol est suffisamment grand pour que les particules générées avec une vitesse élevée aient le temps de ralentir par l'action du frottement de l'air, limitant ainsi leur dépôt par choc sur les parois du moyen complémentaire. Ce moyen complémentaire permet également de concentrer l'aérosol durant la phase expiratoire du patient, augmentant ainsi la quantité de principe actif qu'inhale le sujet à chaque inspiration et augmentant ainsi le débit du système.

L'invention permet donc d'augmenter non seulement le rendement du système de génération d'aérosol mais également son débit.

Ces caractéristiques et d'autres encore ressortiront bien de la suite de la description.
- La figure 1 illustre le principe de base de l'invention .
- Les figures 2.1 et 2.2 sont des vues à caractère schématique de la configuration de la zone de stockage (ZS).

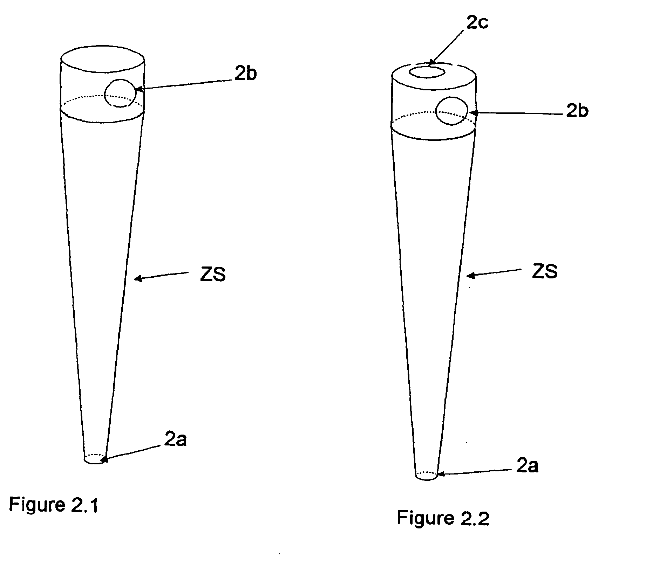

La configuration de la zone de stockage (ZS) qui est illustrée à la figure 2.1 ne fait pas partie de la présente invention.

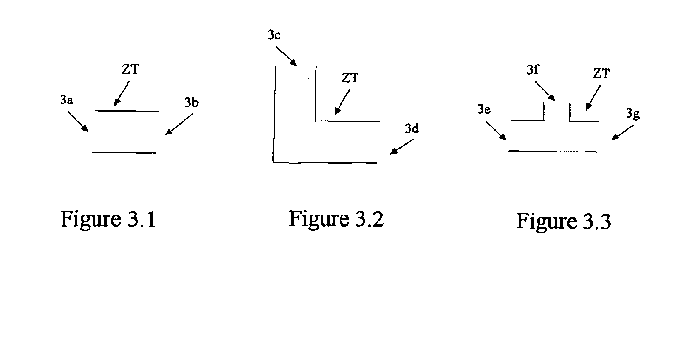

- Les figures 3.1, 3.2, 3.3 sont des vues à caractère schématique illustrant des configurations de la zone de transport (ZT) entre le dispositif de transfert d'aérosol et le patient.

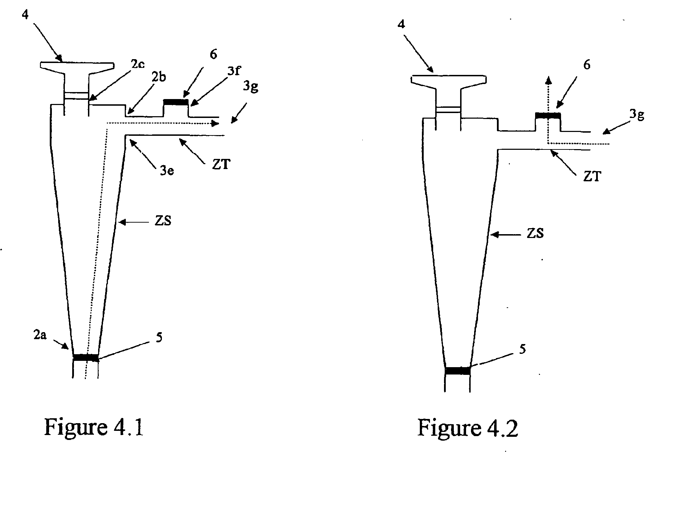

- Les figures 4.1 et 4.2 sont des vues du dispositif de transfert d'aérosol dans le cas de son utilisation avec un générateur d'aérosol à membrane vibrante notamment pour patient ambulatoire.

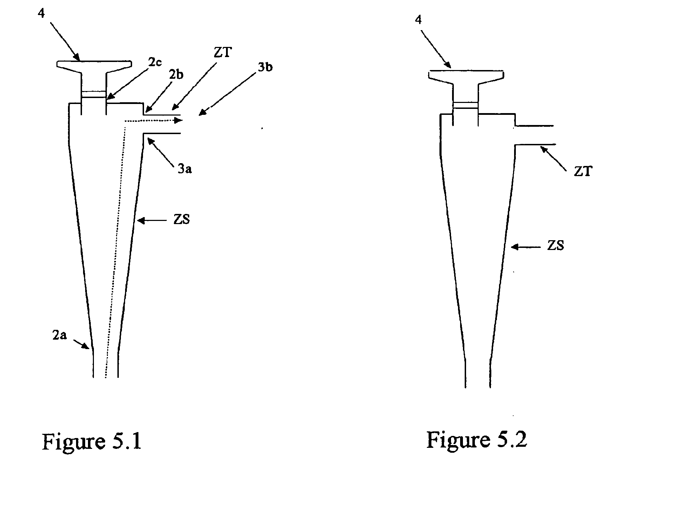

- Les figures 5.1 et 5.2 sont des vues du dispositif de transfert d'aérosol dans le cas de son utilisation avec un générateur d'aérosol dans le cadre d'une ventilation mécanique du patient.

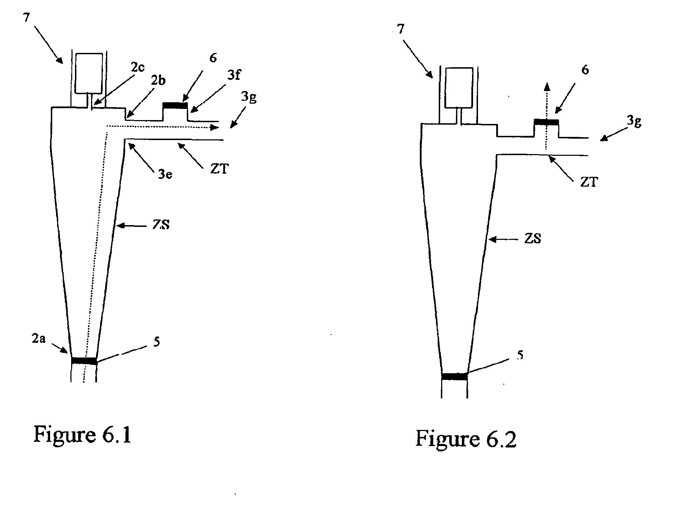

- Les figures 6.1 et 6.2 sont des vues du dispositif de transfert d'aérosol dans le cas de son utilisation avec un générateur d'aérosol flacon doseur pressurisé dans une première mise en oeuvre.

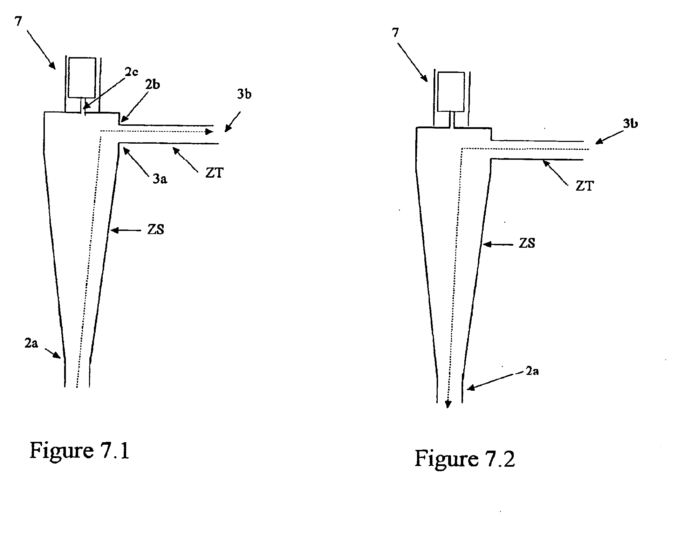

- Les figures 7.1 et 7.2 sont des vues du dispositif de transfert d'aérosol dans le cas de son utilisation avec un générateur d'aérosol flacon doseur pressurisé dans une autre mise en oeuvre.

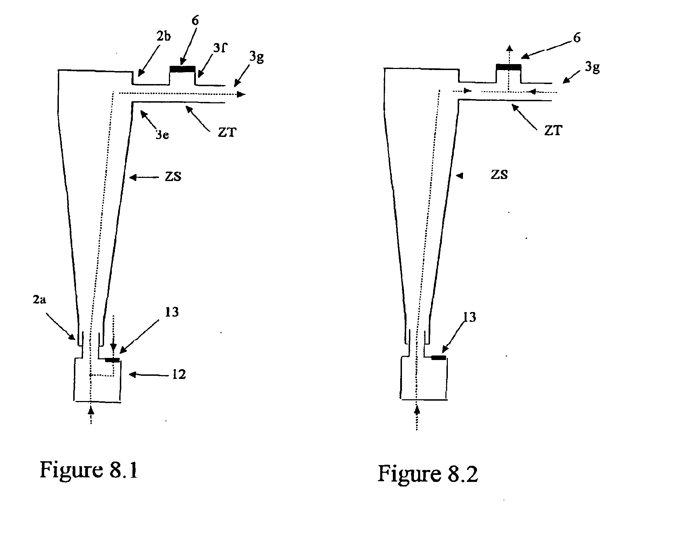

- Les figures 8.1 et 8.2 sont des vues du dispositif de transfert d'aérosol dans le cas de son utilisation avec un générateur d'aérosol pneumatique ou ultrasonique valvé avec prise d'air additionnel ou avec venturi actif. Ce mode de réalisation ne fait pas partie de la présente invention.

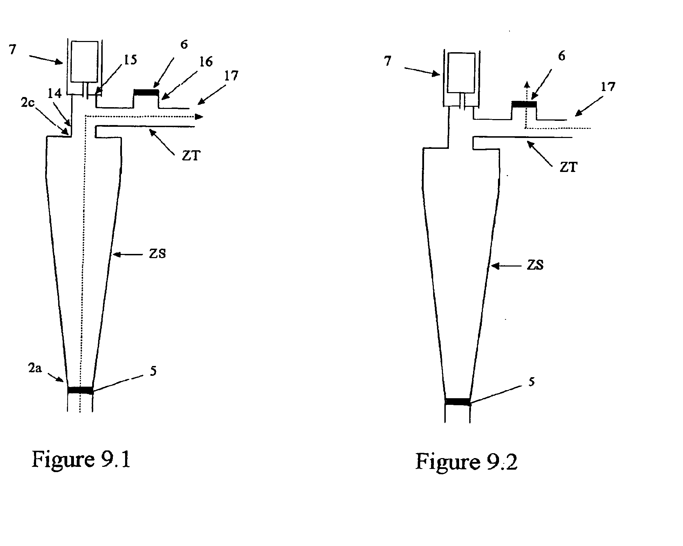

- Les figures 9.1 et 9.2 sont des vues du dispositif de transfert d'aérosol dans le cas de son utilisation avec un générateur d'aérosol flacon doseur pressurisé selon une autre mise en oeuvre. Ce mode de réalisation ne fait pas partie de la présente invention.

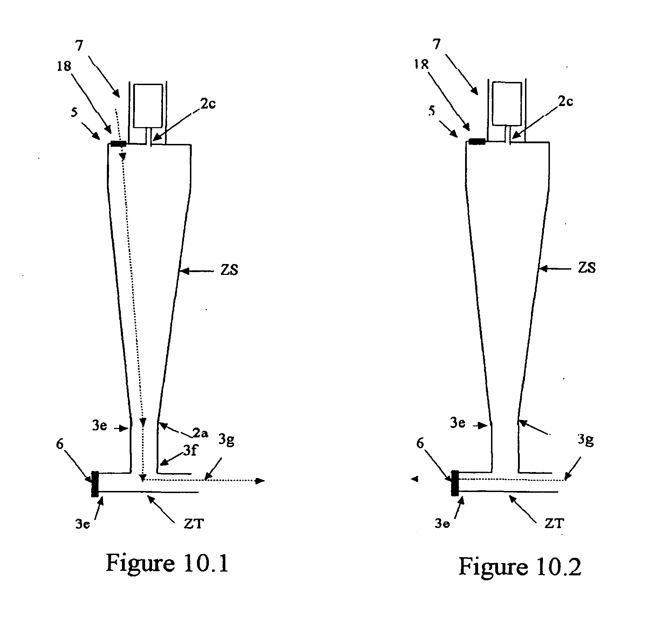

- Les figures 10.1 et 10.2 sont des vues du dispositif de transfert d'aérosol dans le cas de son utilisation avec un générateur d'aérosol flacon doseur pressurisé selon une autre mise en oeuvre. Ce mode de réalisation ne fait pas partie de la présente invention.

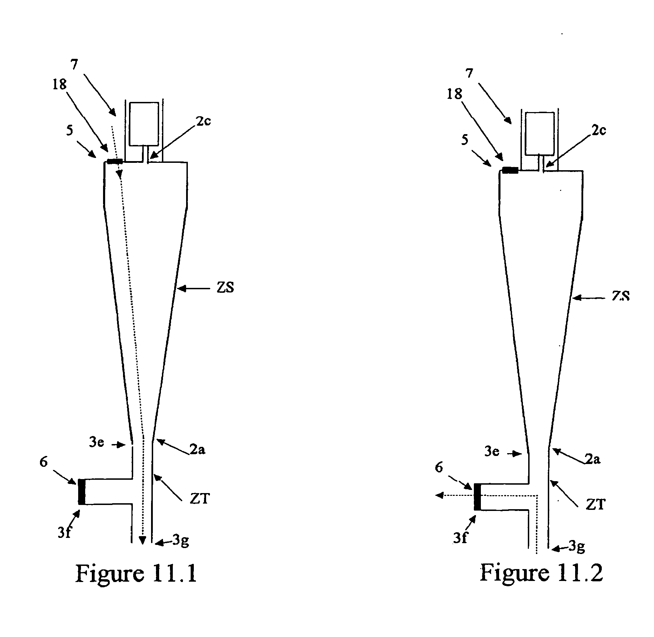

- Les figures 11.1 et 11.2 sont des vues du dispositif de transfert d'aérosol dans le cas de son utilisation pour patient en position allongée (patient alité) avec un générateur d'aérosol flacon doseur pressurisé. Ce mode de réalisation ne fait pas partie de la présente invention.

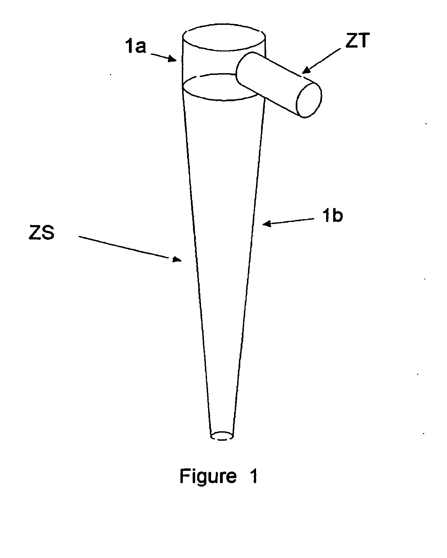

Les systèmes de génération d'aérosol médicaux sont composés d'un générateur d'aérosol à proprement dit et d'une interface entre le générateur d'aérosol et le patient. Le générateur d'aérosol est la source de génération de l'aérosol. L'interface patient - générateur d'aérosol permet le transport de l'aérosol, depuis le générateur vers le patient (exemples : masque, embout buccal, embout narinaire, circuit de ventilateur mécanique, sonde d'intubation, sonde trachéale...). La zone intermédiaire entre la zone de génération de l'aérosol et la zone de transport vers le patient (ZT) est appelée zone de stockage (ZS) (Figure 1).

Cette zone peut être représentée physiquement par le dispositif de transfert d'aérosol. Ce dispositif de transfert d'aérosol, référencé dans son ensemble par (ZS), est destiné à recevoir l'aérosol généré par le générateur d'aérosol, puis par l'intermédiaire de l'interface patient-générateur à le transporter hors du système de génération d'aérosol.

Ce dispositif de transfert d'aérosol est profilé et disposé dans un plan vertical. Il a une hauteur minimale de 6 cm. Sa section peut être de forme quelconque et il peut être composé de plusieurs sections différentes. Il est vertical et de grande longueur. Il présente un col cylindrique dans sa partie supérieure (1a) puis une configuration conique sans fond dans sa partie inférieure (1b) (Figure 1) (pas de surface plane dans son extrémité basse afin de limiter le dépôt de l'aérosol par sédimentation).

Contrairement aux chambres de nébulisation des générateurs d'aérosol de type nébuliseurs pneumatiques ou nébuliseurs ultrasoniques, l'aérosol déposé sur les parois du dispositif de transfert n'est pas recyclé sous forme liquide pour être de nouveau généré sous forme aérosol.

Le dispositif de transfert d'aérosol qui est utilisé dans la présente invention, figure 2.2, contient trois ouvertures (2a), (2b) et (2c) mettant en relation le volume contenu à l'intérieur de la zone de stockage (ZS) avec le volume extérieur à la zone de stockage (ZS).

Dans une des configurations de la zone de stockage qui ne fait pas partie de la présente invention, ces ouvertures peuvent être au nombre de deux, figure 2.1, ouvertures en (2a) et (2b).

Ces ouvertures sont destinées à recevoir au minimum par l'une d'elles (2b) une interface patient-dispositif de transfert d'aérosol. L'autre ouverture (2a) permet au minimum le passage de l'air extérieur à travers le dispositif vers l'interface patient-dispositif de transfert. L'ouverture (2c) permet la projection de l'aérosol par le générateur d'aérosol dans le dispositif. Dans la mise en oeuvre de l'invention, l'ouverture (2a) permettant le passage de l'air depuis l'extérieur de la zone de stockage vers l'intérieur de la zone de stockage doit le faire dans l'axe de la zone de stockage, c'est-à-dire dans un plan vertical. Cette ouverture (2a) se trouve sur la surface horizontale inférieure de la zone de stockage. Cela permet de limiter les turbulences et les effets de tourbillon à l'origine des pertes de particules. L'ouverture (2b) de sortie de l'aérosol de l'intérieur de la zone de stockage vers le patient se trouve à l'opposé de l'ouverture de pénétration d'air de manière à assurer un transfert total de l'aérosol dans le plan vertical de la zone de stockage. L'ouverture (2c) permettant la projection de l'aérosol par le générateur est réalisée en partie haute de la zone de stockage en alignement axial avec l'ouverture (2a) d'entrée d'air avec une projection verticale de l'aérosol dans la zone de stockage.

L'interface entre le dispositif de transfert d'aérosol et le patient contenir au minimum deux ouvertures. Une des ouvertures est obligatoirement destinée à être connectée avec le dispositif de transfert d'aérosol. Les autres ouvertures peuvent être connectées au patient, à des valves et toutes autres sortes de connexions actives ou passives utiles à la séance d'aérosol. Dans une des configurations de l'interface entre le dispositif de transfert d'aérosol et le patient, elle peut être un cylindre ouvert à chaque extrémité, figure 3.1, ouvertures en (3a) et (3b), une pièce en forme de « L » à section circulaire ouverte à chaque extrémité, figure 3.2, ouvertures en (3c) et (3d), une pièce en forme de « T » à section circulaire ouverte à chaque extrémité, figure 3.3, ouvertures en (3e), (3f) et (3g).

A partir de ce principe, différentes configurations du dispositif de transfert d'aérosols peuvent être envisagées, l'aérosol pouvant être à base de liquide ou de poudre selon les configurations.

Une première configuration du dispositif de transfert est illustrée aux figures 4.1 et 4.2, et concerne le cas de son utilisation avec un générateur d'aérosol à membrane vibrante notamment pour patient ambulatoire. Dans cette configuration, la zone de stockage (ZS) possède trois ouvertures. Une première ouverture (2c) placée sur le haut de la zone de stockage (ZS) est destinée à recevoir un nébuliseur à membrane vibrante (4), une deuxième ouverture (2a) placée sur l'extrémité basse est destinée à recevoir une valve (5), une troisième ouverture (2b) placée sur le coté latéral de la partie supérieure est destinée à recevoir la zone de transport vers le patient (ZT). Celle-ci est une pièce en forme de T à section circulaire possédant trois ouvertures (Fig 4.1). L'ouverture (3e) de la zone (ZT) est connectée à l'ouverture (2b) de la zone de stockage (ZS) et l'ouverture (3g) de la zone (ZT) au patient. La troisième ouverture (3f) située sur la partie supérieure de la zone (ZT) (Fig 4.1) est destinée à recevoir une valve (6) (Fig 4.1). Dans cette configuration, durant la phase inspiratoire (Fig 4.1), la valve (5) est ouverte et la valve (6) est fermée. L'air pénétrant dans la zone (ZS) par l'intermédiaire de la valve (5) traverse la zone de stockage (ZS) de bas en haut pour transporter l'aérosol vers la zone (ZT). L'aérosol est ensuite transporté depuis la zone (ZT) vers la bouche du patient.
Durant la phase expiratoire (Fig 4.2), la valve (5) est fermée et la valve (6) est ouverte. L'air expiré par le patient ne passe pas par la zone de stockage (ZS) mais est expulsé hors de la zone (ZT) par la valve (6). Durant la phase expiratoire, l'aérosol continue à être produit et est stocké dans la zone de stockage (ZS) pour la prochaine inspiration.

Une seconde configuration du dispositif de transfert est illustrée aux figures 5.1 et 5.2, et concerne le cas de son utilisation avec un générateur d'aérosol (membrane vibrante notamment ou flacon doseur pressurisé) dans le cadre de la ventilation mécanique. Dans cette configuration, la zone de stockage (ZS) possède trois ouvertures. Une première ouverture (2c) placée sur le haut de la zone (ZS) (Fig 5.1) est destinée à recevoir un générateur d'aérosol (4), une deuxième ouverture (2a) placée sur l'extrémité basse est destinée à être connectée au circuit inspiratoire du système de ventilation mécanique, une troisième ouverture (2b) placée sur le coté latéral de la partie supérieure est destinée à recevoir la zone de transport (ZT). Celle-ci est une pièce cylindrique possédant deux ouvertures. L'ouverture (3a) de la zone (ZT) est connectée à l'ouverture (2b) de la zone de stockage (ZS) et l'ouverture (3b) de la zone (ZT) au circuit inspiratoire du système de ventilation mécanique. Dans cette configuration, le dispositif de transfert est interposé sur le circuit inspiratoire du système de ventilation mécanique.
Dans cette configuration, durant la phase inspiratoire (Fig 5.1), l'air pénétrant dans la zone (ZS) par l'intermédiaire de l'ouverture (2a) traverse la zone de stockage (ZS) de bas en haut pour transporter l'aérosol vers la zone (ZT). L'aérosol est ensuite transporté depuis la zone (ZT) vers le circuit inspiratoire du système de ventilation mécanique.
Durant la phase expiratoire (Fig 5.2), l'air passe par le circuit expiratoire du système de ventilation mécanique et ne traverse donc pas le dispositif de transfert de l'aérosol. Durant cette phase expiratoire, l'aérosol continue à être produit et est stocké dans la zone de stockage (ZS) pour la prochaine inspiration.

Une troisième configuration du dispositif de transfert est illustrée aux figures 6.1 et 6.2, et concerne le cas de son utilisation avec un générateur d'aérosol flacon doseur pressurisé pour patient ambulatoire généralement dénommé flacon pressurisé avec valve doseuse nécessitant l'utilisation d'un système valvé, avec la zone (ZT) placée en haut de la zone de stockage (ZS). Dans cette configuration, la zone de stockage (ZS) possède trois ouvertures. Une première ouverture (2c) placée sur le haut de la zone de stockage (ZS) (Fig
6.1) est destinée à recevoir le flacon doseur pressurisé (7), une deuxième ouverture (2a) placée sur l'extrémité basse est destinée à recevoir une valve (5), une troisième ouverture (2b) placée sur le coté latéral de la partie supérieure est destinée à recevoir la zone (ZT). La zone (ZT) est une pièce en forme de T à section circulaire possédant trois ouvertures (Fig 6.1). L'ouverture (3e) de la zone (ZT) est connectée à l'ouverture (2b) de la zone de stockage (ZS) et l'ouverture (3g) de la zone (ZT) au patient. La troisième ouverture (3f) située sur la partie supérieure de la zone (ZT) est destinée à recevoir une valve (6). Dans cette configuration, durant la phase inspiratoire (Fig 6.1), la valve (5) est ouverte et la valve (6) est fermée. L'air pénétrant dans la zone de stockage (ZS) par l'intermédiaire de la valve (5) traverse la zone de stockage (ZS) de bas en haut pour transporter l'aérosol vers la zone (ZT). L'aérosol est ensuite transporté depuis la zone (ZT) vers la bouche du patient.
Durant la phase expiratoire (Fig 6.2), la valve (5) est fermée et la valve (6) est ouverte. L'air expiré par le patient ne passe pas par la zone (ZS) mais est expulsé hors de la zone (ZT) par la valve (6), l'aérosol reste stocké dans la zone (ZS) pour la prochaine inspiration.

Une quatrième configuration du dispositif de transfert est illustrée aux figures 7.1 et 7.2, et concerne le cas de son utilisation avec un générateur d'aérosol flacon doseur pressurisé pour patients ambulatoires ne nécessitant pas l'utilisation d'un système valvé. Dans cette configuration, la zone (ZS) possède trois ouvertures. Une première ouverture (2c) placée sur le haut de la zone (ZS) est destinée à recevoir le flacon doseur pressurisé (7), une deuxième ouverture (2a) placée sur l'extrémité basse, une troisième ouverture (2b) placée sur le côté latéral de la partie supérieure est destinée à recevoir la zone (ZT). La zone (ZT) est une pièce cylindrique possédant deux ouvertures (Fig 7.1). L'ouverture (3a) de la zone (ZT) est connectée à l'ouverture (2b) de la zone (ZS) et l'ouverture (3b) de la zone (ZT) au patient. Durant la phase inspiratoire, l'air pénétrant dans la zone de stockage (ZS) par l'intermédiaire de l'ouverture (2a) traverse la zone (ZS) de bas en haut pour transporter l'aérosol vers la zone (ZT). L'aérosol est ensuite transporté depuis la zone (ZT) vers le patient. Dans ces conditions, le patient devra nécessairement faire une inspiration avec un volume suffisamment important après la production de la bouffée d'aérosol pour inspirer la totalité de l'aérosol stocké dans la zone (ZS).
Durant la phase expiratoire (Fig 7.2), l'air exhalé par le patient traverse la zone (ZS) de haut en bas.

Une cinquième configuration du dispositif de transfert qui ne fait pas partie de la présente invention est illustrée aux figures 8.1 et 8.2, et concerne le cas de son utilisation avec un générateur d'aérosol pneumatique valvé avec venturi actif ou avec un nébuliseur ultrasonique valvé de préférence ventilé. Dans cette configuration qui ne fait pas partie de la présente invention, la zone (ZS) possède deux ouvertures. Une première ouverture (2a) placée sur l'extrémité basse de la zone (ZS) est destinée à recevoir le nébuliseur pneumatique ou ultrasonique (12) valvé en (13), une deuxième ouverture (2b) placée sur le coté latéral de la partie supérieure est destinée à recevoir la zone (ZT). La zone (ZT) est une pièce en forme de T à section circulaire possédant trois ouvertures. L'ouverture (3e) de la zone (ZT) est connectée à l'ouverture (2b) de la zone (ZS) et l'ouverture (3g) de la zone (ZT) au patient. La troisième ouverture située sur la partie supérieure de la zone (ZT) (3f) est destinée à recevoir une valve (6). Dans cette configuration, durant la phase inspiratoire et plus précisément lorsque le débit inspiratoire du patient est supérieur au débit d'air du nébuliseur, la valve (13) est ouverte et la valve (6) est fermée. L'air pénétrant dans la zone (ZS) par l'intermédiaire de la valve (13) et l'air du nébuliseur traversent la zone (ZS) de bas en haut pour transporter l'aérosol vers la zone (ZT). L'aérosol est ensuite transporté depuis la zone (ZT) vers la bouche du patient.
Durant la phase expiratoire (Fig 8.2) et plus précisément lorsque le débit inspiratoire du patient est inférieur au débit d'air, la valve (13) est fermée et la valve (6) est ouverte. L'air expiré par le patient ne passe pas par la zone (ZS) mais est expulsé hors de la zone (ZT) par la valve (6). Durant la phase expiratoire, l'aérosol continue à être produit par l'air du nébuliseur. L'air du nébuliseur contenant des particules d'aérosol traverse la zone (ZS) vers la zone (ZT), puis est expulsé hors de la zone (ZT) par la valve (6). Lors de ce transport dans la zone (ZS), les particules, sous l'effet de leur poids, vont sédimenter dans l'air en écoulement. L'air dirigé vers la zone (ZT) va s'appauvrir en particules.
L'aérosol stocké dans la zone (ZS) sera inhalé à la prochaine inspiration.

Une sixième configuration du dispositif de transfert qui ne fait pas partie de la présente invention est illustrée aux figures 9.1 et 9.2, et concerne le cas de son utilisation avec un générateur d'aérosol flacon doseur pressurisé pour patient ambulatoire nécessitant l'utilisation d'un système valvé avec la zone (ZT) placée en haut de la zone (ZS). Dans cette configuration qui ne fait pas partie de la présente invention, la zone (ZS) possède deux ouvertures. Une première ouverture (2c) placée sur le haut de la zone (ZS) est destinée à recevoir la zone (ZT), une deuxième ouverture (2a) placée sur l'extrémité basse est destinée à recevoir une valve (5). La zone (ZT) est une pièce à section circulaire possédant quatre ouvertures. L'ouverture (14) de la zone (ZT) est connectée à l'ouverture (2c) de la zone (ZS), l'ouverture (15) de la zone (ZT) reçoit le générateur d'aérosol (7), l'ouverture (16) de la zone (ZT) est destinée à recevoir une valve (6) et l'ouverture (17) de la zone (ZT) est connectée au patient. Dans cette configuration, durant la phase inspiratoire, la valve (5) est ouverte et la valve (6) est fermée. L'air pénétrant dans la zone (ZS) par l'intermédiaire de la valve (5) traverse la zone (ZS) de bas en haut pour transporter l'aérosol vers la zone (ZT). L'aérosol est ensuite transporté depuis la zone (ZT) vers la bouche du patient.
Durant la phase expiratoire (Fig 9.2), la valve (5) est fermée et la valve (6) est ouverte. L'air expiré par le patient ne passe pas par la zone (6) mais est expulsé hors de la zone (ZT) par la valve (6), l'aérosol reste stocké dans la zone (ZS) pour la prochaine inspiration.

Une septième configuration du dispositif de transfert qui ne fait pas partie de la présente invention est illustrée aux figures 10.1 et 10.2, et concerne le cas de son utilisation avec un générateur d'aérosol flacon doseur pressurisé pour patient en position assise ou debout nécessitant l'utilisation d'un système valvé avec la zone (ZT) placée en bas de la zone de stockage (ZS). Dans cette configuration, la zone (ZS) possède trois ouvertures. Une première ouverture (2c) placée sur le haut de la zone (ZS) est destinée à recevoir le flacon doseur pressurisé (7), une deuxième ouverture (2a) placée sur l'extrémité basse est destinée à recevoir la zone (ZT), une troisième ouverture (18) placée sur la partie horizontale supérieure est destinée à recevoir une valve (5). La zone (ZT) est une pièce en forme de T à section circulaire possédant trois ouvertures. L'ouverture (3e) de la zone (ZT) est connectée à l'ouverture (2a) de la zone (ZS) et l'ouverture (3g) de la zone (ZT) au patient. La troisième ouverture (3f) de la zone (ZT) est destinée à recevoir une valve (6). Dans cette configuration, durant la phase inspiratoire (Fig 10.1), la valve (5) est ouverte et la valve (6) est fermée. L'air pénétrant dans la zone (ZS) par l'intermédiaire de la valve (5) traverse la zone (ZS) de haut en bas pour transporter l'aérosol vers la zone (ZT). L'aérosol est ensuite transporté depuis la zone (ZT) vers la bouche du patient.
Durant la phase expiratoire (Fig 10.2), la valve (5) est fermée et la valve (6) est ouverte. L'air expiré par le patient ne passe pas par la zone (ZS) mais est expulsé hors de la zone (ZT) par la valve (6), l'aérosol reste stocké dans la zone (ZS) pour la prochaine inspiration.

Une huitième configuration du dispositif de transfert qui ne fait pas partie de la présente invention est illustrée aux figures 11.1 et 11.2, et concerne le cas de son utilisation avec un générateur d'aérosol flacon doseur pressurisé pour patient en position allongée nécessitant l'utilisation d'un système valvé avec la zone (ZT) placée en bas de la zone de stockage (ZS). Dans cette configuration, la zone (ZS) possède trois ouvertures. Une première ouverture (2c) placée sur le haut de la zone (ZS) est destinée à recevoir le flacon doseur pressurisé (7), une deuxième ouverture (2a) placée sur l'extrémité basse est destinée à recevoir la zone (ZT), une troisième ouverture (18) placée sur la partie horizontale supérieure est destinée à recevoir une valve (5). La zone (ZT) est une pièce en forme de T à section circulaire possédant trois ouvertures. L'ouverture (3e) de la zone (ZT) est connectée à l'ouverture (2a) de la zone (ZS) et l'ouverture (3g) de la zone (ZT) au patient. La troisième ouverture (3f) de la zone (ZT) est destinée à recevoir une valve (6). Dans cette configuration, durant la phase inspiratoire (Fig 11.1), la valve (5) est ouverte et la valve (6) est fermée. L'air pénétrant dans la zone (ZS) par l'intermédiaire de la valve (5) traverse la zone (ZS) de haut en bas pour transporter l'aérosol vers la zone (ZT). L'aérosol est ensuite transporté depuis la zone (ZT) vers la bouche du patient.
Durant la phase expiratoire (Fig 11.2), la valve (5) est fermée et la valve (6) est ouverte. L'air expiré par le patient ne passe pas par la zone (ZS) mais est expulsé hors de la zone (ZT) par la valve (6), l'aérosol reste stocké dans la zone (ZS) pour la prochaine inspiration.

Dans les configurations précitées, la disposition des ouvertures et valves peuvent varier en position, les figures ayant été décrites et citées à titre d'exemple.

Les configurations décrites précédemment, apparaissent extrêmement avantageuses, car selon les tests effectués, il a été mesuré que 40 à 90 % de la dose d'aérosol, en fonction des générateurs d'aérosols utilisés, est inhalée par le patient, c'est-à-dire avec des proportions de l'ordre de trois fois de ce qui est connu sur le marché.

Le dispositif interface de stockage qui est utilisé dans la présente invention est facile à réutiliser et à adapter sur des générateurs d'aérosols médicaux. Son nettoyage est aisé également.

Evaluation des performances du dispositif de transfert d'aérosol selon la norme NF-EN13544-1

Pour évaluer les générateurs d'aérosols médicamenteux, la norme européenne EN13544-1 a standardisé un protocole expérimental pour déterminer la masse inhalable de l'aérosol produit par les générateurs d'aérosols médicaux.
La mesure de la masse inhalable consiste à filtrer l'aérosol produit durant la phase inspiratoire dans des conditions standardisées de ventilation de patient. Cette masse filtrée correspond à la masse d'aérosol délivrée dans la bouche du patient. La fraction inhalable est définie par le rapport entre la masse inhalable et la masse de médicament introduite dans le générateur d'aérosol.
Pour évaluer l'effet du dispositif de transfert d'aérosol utilisé dans la présente invention sur les systèmes existant nous avons évalué dans un cas la performance des générateurs d'aérosols sans le dispositif de transfert d'aérosol et dans autre cas la performance de ces mêmes générateurs d'aérosol avec le dispositif de transfert d'aérosol. Un nébuliseur à membrane vibrante notamment (Aeroneb®, Aerogen, USA) était testé dans un cas sans le dispositif de transfert d'aérosol et dans un autre cas avec le dispositif de transfert d'aérosol selon la configuration 1 (Fig 4.1 et fig 4.2). Un flacon pressurisé avec valve doseuse (Flacon doseur de Bricanyl®, Astra Zeneca, Suede) était testé dans un cas avec une chambre d'inhalation (Nebuhaler®, Astra Zeneca, Suede) et dans un autre cas avec le dispositif de transfert d'aérosol selon la configuration 3 (Fig 6.1 et Fig 6.2). Le nébuliseur pneumatique NL9M® (La Diffusion Technique Française, Saint Etienne, France) était testé dans un cas sans le dispositif de transfert d'aérosol et dans un autre cas avec le dispositif de transfert d'aérosol.

| Générateur d'aérosol | Fraction inhalable | Durée de la séance |
|---|---|---|
| NL9M® | 25% | 4min 30 sec |
| NL9M® + dispositif | 40% | 5min |
| Aeroneb® | 25% | 4min 30sec |
| Aeroneb® + dispositif | 91% | 4min 30sec |
| Flacon doseur de Bricanyl® +Nebuhaler® | 14% | -non applicable |
| Flacon doseur de Bricanyl® +Dispositif | 51% | -non applicable |

Tableau 1 : Résultats des performances des générateurs d'aérosols médicaux selon la norme NF EN13544-1

Les résultats montrent que le dispositif de transfert d'aérosol qui est utilisé dans la présente invention permet dans tous les cas d'augmenter la fraction inhalable d'aérosol pour le patient. Le dispositif permet une augmentation de 60 % à 264 % de la performance des générateurs d'aérosol en terme de masse inhalable d'aérosol. Le dispositif n'influençant pas la durée de nébulisation, les résultats démontrent également que le dispositif permet d'augmenter le débit d'aérosol administré au patient. Pour le générateur d'aérosol flacon doseur de Bricanyl®, il n'y a pas de durée de séance car la dose est délivrée instantanément (quelques dixièmes de secondes).

## Revendications

1. Appareil pour l'administration d'un aérosol médicamenteux comprenant:
- un générateur d'aérosol médicamenteux; et
- un dispositif de transfert d'aérosol destiné à recevoir l'aérosol médicamenteux généré par le générateur d'aérosol;
- le dispositif de transfert d'aérosol comprenant un moyen complémentaire profilé et disposé dans un plan vertical constituant une zone de stockage (ZS) de l'aérosol;
- le moyen complémentaire présentant un col cylindrique dans sa partie supérieure (1a) puis une configuration conique sans fond et rétrécie dans sa partie inférieure (1b) pour favoriser le transport de l'aérosol de part et d'autre du moyen complémentaire;
- le moyen complémentaire étant agencé avec une première ouverture (2a) permettant le passage de l'air depuis l'extérieur de la zone de stockage (ZS) vers l'intérieur de celle-ci en étant disposée sur la surface horizontale inférieure dudit moyen pour assurer une pénétration verticale de l'air dans ledit moyen;
- le moyen complémentaire comprenant une seconde ouverture (2b) autorisant la sortie de l'aérosol de l'intérieur de la zone de stockage (ZS) vers le patient et étant disposée à l'opposé de ladite première ouverture (2a) dudit moyen de manière à assurer un transfert total de l'aérosol dans le plan vertical de la zone de stockage (ZS);
- le moyen complémentaire comprenant une troisième ouverture (2c) permettant la projection de l'aérosol par le générateur dans la zone de stockage (ZS) et étant disposée sur la surface horizontale supérieure dudit moyen en alignement axial de l'ouverture (2a) à entrée d'air de manière à assurer une projection verticale de l'aérosol dans la zone de stockage (ZS);
- le générateur d'aérosol étant connectée à la troisième ouverture (2c) du moyen complémentaire pour assurer la projection de l'aérosol dans le plan vertical de la zone de stockage (ZS);
- le moyen complémentaire assurant la fonction de stockage étant d'une hauteur minimale de 6 cm et autorisant une dispersion de l'aérosol projeté dans la totalité du volume sans recyclage de l'aérosol déposé sur les parois;
- ladite zone de stockage (ZS) étant disposée entre le générateur d'aérosol et une interface patient, et reliée à une zone de transport (ZT) de l'aérosol vers le patient;
- le dispositif n'incluant aucune valve sur le trajet de l'aérosol depuis le moyen constitutif de la zone de stockage (ZS) vers le patient.

2. Appareil selon la revendication 1, **caractérisé en ce que** la zone de transport (ZT) consiste en un cylindre ouvert à chaque extrémité.

3. Appareil selon la revendication 1, **caractérisé en ce que** la zone de transport (ZT) consiste en une pièce en forme de L à section circulaire ouverte à chaque extrémité.

4. Appareil selon la revendication 1, **caractérisé en ce que** la zone de transport (ZT) consiste en une pièce une forme en T à section circulaire ouverte à chaque extrémité.

5. Appareil selon l'une des revendications 1 à 4, **caractérisé en ce que** le générateur d'aérosol consiste en un nébuliseur à membrane vibrante.

6. Appareil selon l'une des revendications 1 à 4, **caractérisé en ce que** le générateur d'aérosol consiste en un nébuliseur pneumatique.

7. Appareil selon l'une des revendications 1 à 4, **caractérisé en ce que** le générateur d'aérosol consiste en un nébuliseur ultrasonique.

8. Appareil selon l'une des revendications 1 à 5, **caractérisé en ce que** le générateur d'aérosol consiste en un flacon pressurisé muni d'une valve doseuse.

## Claims

1. Apparatus for the delivery of a medicinal aerosol comprising:
- a generator of medicinal aerosol; and
- an aerosol transfer device intended to receive the medicinal aerosol generated by the aerosol generator;
- the transfer device comprising a complementary shaped means placed in a vertical plane forming a zone (ZS) for storing the aerosol;
- the complementary means having a cylindrical neck in the upper part (1a) thereof and then a bottomless conical configuration narrowed in the lower part (1b) thereof so as to promote the transmission of the aerosol on both sides of the complementary means;
- the complementary means being provided with a first opening (2a), so that the air can pass from the outside of the storage zone (ZS) to the inside thereof, placed on the lower horizontal surface of said means to ensure that the air penetrates vertically into said means;
- the complementary means comprising a second opening (2b), so that the aerosol can depart from the inside of the storage zone (ZS) in the direction of the patient, placed on the opposite side from said first opening (2a) of said means to ensure that the aerosol is fully transferred in the vertical plane of the storage zone (ZS);
- the complementary means comprising a third opening (2c), so that the aerosol can be projected by the generator into the storage zone (ZS), placed on the upper horizontal surface of said means in axial alignment with the air inlet opening (2a) to ensure that the aerosol is projected vertically into the storage zone (ZS);
- the aerosol generator being connected to the third opening (2c) of the complementary means to ensure that the aerosol is projected in the vertical plane of the storage zone (ZS);
- the complementary means fulfilling the storage function being at least 6 cm in height and allowing the projected aerosol to be dispersed into the totality of the volume without recycling the aerosol deposited on the walls;
- said storage zone (ZS) being placed between the aerosol generator and a patient interface, and connected to an aerosol-to-patient transmission zone (ZT);
- the device not including any valves on the path taken by the aerosol from the means forming the storage zone (ZS) to the patient.

2. Apparatus as claimed in claim 1, **characterized in that** the transmission zone (ZT) consists of a cylinder open at each end.

3. Apparatus as claimed in claim 1, **characterized in that** the transmission zone (ZT) consists of an L-shaped part of circular cross-section open at each end.

4. Apparatus as claimed in claim 1, **characterized in that** the transmission zone (ZT) consists of a T-shaped part of circular cross-section open at each end.

5. Apparatus as claimed in one of claims 1 to 4, **characterized in that** the aerosol generator consists of a vibrating membrane nebulizer.

6. Apparatus as claimed in one of claims 1 to 4, **characterized in that** the aerosol generator consists of a pneumatic nebulizer.

7. Apparatus as claimed in one of claims 1 to 4, **characterized in that** the aerosol generator consists of an ultrasonic nebulizer.

8. Apparatus as claimed in one of claims 1 to 5, **characterized in that** the aerosol generator consists of a pressurized bottle provided with a dosing valve.

## Patentansprüche

1. Gerät für die Verabreichung eines Arzneimittel-Aerosols mit:
- einem Arzneimittel-Aerosolgenerator; und
- einer Aerosol-Transfervorrichtung für die Aufnahme des vom Aerosolgenerator erzeugten Arzneimittel-Aerosols;
- wobei die Aerosol-Transfervorrichtung eine ergänzende profilierte Einrichtung umfasst, die als Speicherzone (ZS) des Aerosols in einer vertikalen Ebene angeordnet ist;
- wobei die ergänzende Einrichtung einen zylindrischen Hals in seinem oberen Teil (1a) und dann eine bodenlose konische Form aufweist, die in ihrem unteren Teil (1b) verengt ist, um den Aerosoltransport beiderseits der ergänzenden Einrichtung zu begünstigen;
- wobei die ergänzende Einrichtung mit einer ersten Öffnung (2a) ausgebildet ist, die den Luftdurchlass von der Außenseite der Speicherzone (ZS) in ihr Inneres ermöglicht und dabei an der unteren waagrechten Fläche der besagten Einrichtung angeordnet ist, um ein vertikales Eindringen der Luft in die besagte Einrichtung zu gewährleisten;
- wobei die ergänzende Einrichtung eine zweite Öffnung (2b) umfasst, die den Austritt des Aerosols vom Inneren der Speicherzone (ZS) zum Patienten ermöglicht und dabei in Opposition zur besagten ersten Öffnung (2a) der besagten Einrichtung angeordnet ist, um für den völligen Transfer des Aerosols in die vertikale Ebene der Speicherzone (ZS) zu sorgen;
- wobei die ergänzende Einrichtung eine dritte Öffnung (2c) umfasst, die das Sprühen des Aerosols durch den Generator in die Speicherzone (ZS) ermöglicht und an der oberen waagrechten Fläche der besagten Einrichtung in axialer Ausrichtung zur Lufteinlassöffnung (2a) angeordnet ist, um für ein vertikales Sprühen des Aerosols in die Speicherzone (ZS) zu sorgen;
- wobei der Aerosolgenerator mit der dritten Öffnung (2c) der ergänzenden Einrichtung verbunden ist, um für das Sprühen des Aerosols in die vertikale Ebene der Speicherzone (ZS) zu sorgen;
- wobei die für die Speicherung sorgende ergänzende Einrichtung mindestens 6 cm hoch ist und eine Dispersion des versprühten Aerosols in das gesamte Volumen ohne Rückführung des an den Wänden abgesetzten Aerosols ermöglicht;
- wobei die Speicherzone (ZS) zwischen dem Aerosolgenerator und einer Patientenschnittstelle angeordnet und mit einer Transportzone (ZT) des Aerosols zum Patienten verbunden ist;
- wobei die Vorrichtung auf dem Weg des Aerosols von der die Speicherzone (ZS) bildenden Einrichtung zum Patienten kein Ventil umfasst.

2. Gerät nach Anspruch 1, **dadurch gekennzeichnet, dass** die Transportzone (ZT) aus einem an jedem Ende offenen Zylinder besteht.

3. Gerät nach Anspruch 1, **dadurch gekennzeichnet, dass** die Transportzone (ZT) aus einem an jedem Ende offenen L-förmigen Teil mit kreisförmigem Querschnitt besteht.

4. Gerät nach Anspruch 1, **dadurch gekennzeichnet, dass** die Transportzone (ZT) aus einem an jedem Ende offenen T-förmigen Teil mit kreisförmigem Querschnitt besteht.

5. Gerät nach einem der Ansprüche 1-4, **dadurch gekennzeichnet, dass** der Aerosolgenerator aus einem Schwingmembran Vernebler besteht.

6. Gerät nach einem der Ansprüche 1 - 4, **dadurch gekennzeichnet, dass** der Aerosolgenerator aus einem Druckluftvernebler besteht.

7. Gerät nach einem der Ansprüche 1-4, **dadurch gekennzeichnet, dass** der Aerosolgenerator aus einem Ultraschallvernebler besteht.

8. Gerät nach einem der Ansprüche 1-5, **dadurch gekennzeichnet, dass** der Aerosolgenerator aus einer Druckflasche mit Dosierventil besteht.
